# EUROPEAN PATENT SPECIFICATION

(11) **EP 3 456 348 B1**
(45) Date of publication and mention of the grant of the patent: **26.03.2025**
(21) Application number: 18187826.5
(22) Date of filing: 11.09.2007
(51) Int. Cl.: A61K 39/145, C07K 14/10

(54) **MAKING INFLUENZA VIRUS VACCINES WITHOUT USING EGGS**
HERSTELLUNG VON INFLUENZA-VIRUS-IMPFSTOFFEN OHNE VERWENDUNG VON EIERN
FABRICATION DE VACCINS CONTRE LE VIRUS DE LA GRIPPE SANS UTILISER D' OEUFS

(30) Priority: 11.09.2006 US 84372006 P; 18.06.2007 US 93627907 P
(43) Date of publication of application: 20.03.2019
(62) Divisional of application: 12156030.4
(73) Proprietor: Seqirus UK Limited, Maidenhead, Berkshire SL6 8AA (GB)
(72) Inventor: TSAI, Theodore F., Cambridge, MA 02139 (US); TRUSHEIM, Heidi, 35041 Marburg (DE)
(74) Representative: Carpmaels & Ransford LLP

(56) References cited:
- WO-A1-2006/027698
- US-A1- 2005 118 698
- MERTEN O W ET AL: "PRODUCTION OF INFLUENZA VIRUS IN CELL CULTURES FOR VACCINE PREPARATION", ADVANCES IN EXPERIMENTAL MEDICINE AND BIOLOGY, vol. 397, 1996, pages 141 - 151, XP000982880, ISSN: 0065-2598
- MEDEMA JEROEN K ET AL: "Modeling pandemic preparedness scenarios: health economic implications of enhanced pandemic vaccine supply.", VIRUS RESEARCH, vol. 103, no. 1-2, July 2004 (2004-07-01), pages 9 - 15, XP002477504, ISSN: 0168-1702
- MERTEN O-W ET AL: "PRODUCTION OF INFLUENZA VIRUS IN SERUM-FREE MAMMALIAN CELL CULTURES", DEVELOPMENTS IN BIOLOGICAL STANDARDIZATION, KARGER, BASEL, CH, vol. 98, 1 January 1997 (1997-01-01), pages 23 - 37, XP000909398, ISSN: 0301-5149

## Description

### TECHNICAL FIELD

This invention is in the field of manufacturing vaccines for protecting against influenza virus.

### BACKGROUND ART

The current process for preparing seasonal vaccines against human influenza virus infection involves the following steps [1,2]: (a) isolation of circulating virus strains; (b) antigenic and genetic analysis of isolated viruses; (c) selection of viral strains for use during the coming season; (d) preparation of high-growth seed strains by reassortment or the use of reverse genetics; (e) release of seed strains to vaccine manufacturers; (f) evaluation by the manufacturers of the strains' suitability for industrial production; and (g) growth of the seed strains to produce virus from which vaccines are then manufactured.

Steps (a) to (e) of this process are performed by the FDA and government-approved international influenza centres, typically under the auspices of the World Health Organisation; steps (f) and (g) are performed by the manufacturers themselves.

Step (d) transitions a virus from one that is naturally adapted for infecting humans into one that will grow to high titers under industrial growth conditions. For influenza A virus, this step typically involves creating a 6:2 reassortant strain that includes the HA- and NA-encoding genome segments from the strains selected in (c) and the remaining six genome segments from a strain that grows efficiently in chicken eggs, and this strain is usually A/PR/8/34. The reassortment procedure is then followed by repeated passaging of the strain in embryonated eggs to allow for egg adaptation and growth enhancement. For influenza B virus, prototype strains with good growth characteristics are usually obtained by direct and repeated passaging in embryonated eggs without attempting to generate reassortants.

Thus the steps performed prior to release to vaccine manufacturers involve passaging influenza virus through eggs. Even if the viruses are grown by a manufacturer in step (g) on a cell substrate, rather than on eggs, the virus will still have been passaged through eggs at some stage between isolation from in step (a) and receipt by a manufacturer in step (e).

For instance, step (a) involves exposing a substrate to a patient sample, such that any virus in the sample will infect the substrate. The substrate can then amplify the amount of virus present, and the amplified viruses are then available for further study. This step may take place in eggs or in mammalian cells. Cells known for use in primary isolation include MRC-5 cells [3], Vero cells [4,5], MDCK cells [6], HepG2 cells [7], LLC-MK2 cells [8], *etc.* In general, though, chicken eggs continue to be used to isolate reference strains for the manufacture of influenza vaccine. The use of eggs is so important to current procedures that in the 2003-04 season the FDA rejected use of the most appropriate H3N2 strain (A/Fujian/411/2002) because it had not originally been isolated in eggs [2,9] and no antigenically-similar egg-isolated strains were available.

It has previously been proposed to remove the use of eggs from various stages of influenza virus manufacture.

Reference 10 proposes that vaccines should be grown in cell culture using either a (i) a high growth strain of a passaged clinical isolate or (ii) a reassortant derived from at least one naturally-occurring mammalian influenza virus strain, provided that the isolate or reassortant has not been passaged in avian eggs. Thus the process described in reference 10 begins with a seed virus that has already been selected or manipulated for growth in the cell culture of choice.

Reference 11 compares viruses passaged through eggs with those passaged through MDCK cells, but specifically selects the former for vaccine manufacture.

Reference 12 suggests that seed viruses for pandemic influenza vaccines could be prepared by propagating the pandemic strain directly onto mammalian cell culture instead of via embryonated eggs, but notes that egg passaging was obligatory for inter-pandemic manufacture. The reason for this obligatory egg passaging is that it has been believed to act as a 'filter' for adventitious agents: regulatory agencies have accepted that a series of passages in an avian system, between the original clinical isolation from a human and the final vaccine for administration to a human, will prevent mammalian-type adventitious agents from co-replicating with the influenza virus.

The invention aims to provide further procedures useful in manufacturing influenza vaccines, in which the use of eggs is reduced, and preferably is avoided altogether. In one particular aspect, the invention aims to provide further and improved procedures useful in influenza virus isolation.

### DISCLOSURE OF THE INVENTION

Although it has previously been proposed to remove the use of eggs from various stages of influenza virus manufacture, the invention differs from these proposals in various aspects.

The invention provides a process for manufacturing a vaccine comprising steps of: (i) isolating an influenza virus from a patient sample, comprising a step in which the patient sample is incubated with a MDCK cell, wherein the MDCK cell is growing in a serum-free suspension culture, and (ii) manufacturing a vaccine from the influenza virus isolated in step (i), wherein the influenza virus is not grown in eggs before being exposed to MDCK cells, and wherein after isolation, the virus is not subsequently grown in eggs.

In a further aspect, the invention provides a process for preparing an influenza virus antigen for use in a vaccine, comprising steps of: (i) receiving an influenza virus isolated from a patient sample by a method comprising a step in which the patient sample is incubated with a MDCK cell, wherein the MDCK cell is growing in a serum-free suspension culture; (ii) infecting a cell line with this influenza virus; and (iii) culturing the infected cells from step (ii) in order to produce influenza virus, wherein the influenza virus is not grown in eggs before being exposed to MDCK cells, and wherein after isolation, the virus is not subsequently grown in eggs.

### Preparation of seed viruses

A first aspect of the disclosure provides a process for preparing an influenza seed virus for vaccine manufacture, comprising steps of: (i) infecting a cell line with an influenza virus obtained either directly from a patient or from a primary isolate; (ii) passaging the virus from the infected cell line obtained in step (i) at least once; and (iii) culturing the infected cells from step (ii) in order to produce influenza virus. Influenza virus purified from the culture of step (iii) can be used as the seed virus.

In contrast to reference 12, the influenza virus used in step (i) is either an influenza B virus or is a non-pandemic influenza A virus *i.e*. at the current time is a H1N1 or H3N2 strain of influenza A.

None of steps (i), (ii) or (iii) involves growth or passaging of the virus in eggs. Preferably at least two of the steps, and ideally all three steps, will take place in the same cell type *e.g.* all in MDCK cells.

The passaging in step (ii) will typically involve: allowing the influenza virus to replicate in cell culture; collecting replicated virus *e.g*. from the culture supernatant; and transferring the collected replicated virus to an uninfected cell culture. This process can be repeated. After at least one passage, the virus is allowed to replicate in step (iii) and virus is collected, but the virus is used as a seed virus rather than being transferred to an uninfected culture for further passaging.

The cell line used with the first aspect is preferably not a human cell line. By avoiding the use of human cells, the 'filtering' of adventitious agents can be maintained even without the use of eggs. Because of the close relationship to humans, the cell line is also preferably not a primate cell line *e.g.* it is not a Vero cell line (derived from monkey kidney). Reference 10 proposes the use of Vero cells during vaccine manufacture but these cells are permissive to many human viruses, and so any further human viruses that are present in the influenza virus sample used in step (i) will be able to grow in parallel with the influenza virus, leading to contamination of the eventual seed virus.

A preferred cell line for use with the first aspect of the disclosure is a canine cell line, such as the MDCK cell line (Madin Darby canine kidney), contrary to the specific teaching of reference 10. Further details of MDCK cells are given below. It has now been found that MDCK cells exhibit a 'filtering' effect against adventitious agents that is equivalent to that achieved by avian eggs. Based on this finding, therefore, MDCK cells can be used in place of eggs without increasing regulatory risk and, despite the reports in reference 13 that egg passaging on influenza virus conveys a growth advantage during MDCK culture, growth in eggs is avoided.

Reference 14 discloses that influenza strains isolated from human patients, without any passages in eggs or cell culture, can grow efficiently in MDCK cell cultures, including in serum-free cultures. Thus the infection in step (i) may use an influenza virus from a clinical sample (*e.g*. from a pharyngeal swab, *etc*.) obtained directly from a patient, or it may use a virus which has already been subjected to primary isolation. In some circumstances, the primary isolation prior to step (i) may have taken place in eggs, but preferred primary isolates for use with the invention are those which were obtained without the use of eggs *e.g.* in mammalian cells. Cells known for use in primary isolation include, but are not limited to, MRC-5 cells [3], Vero cells [4,5], MDCK cells [6], HepG2 cells [7], LLC-MK2 cells [8], *etc.*

Where the process uses primary isolates in step (i), it is preferred that primary isolation should have taken place in the same cell type as steps (i) to (iii). MDCK is already known to be suitable for both primary isolation, passaging and growth of influenza viruses, but improvements in MDCK isolation are described below.

In order to maximise knowledge of the history of an influenza virus isolate, it is preferred to use a virus obtained directly from a clinical isolate rather than to use primary isolates. Current influenza surveillance systems involve primary isolation in hospitals, with strains of interest being sent on to national and international influenza centres. In addition to using a patient sample for primary isolation, it is common for a portion to be set aside and stored (*e.g*. by freezing) such that it is possible to return to the original material for re-isolation. Where such a stored sample is available then it can be used in step (i) in place of the primary isolate.

If the history of an isolate is unclear, and in particular when starting with a virus that is not directly from a clinical sample, it is possible to use reverse genetics between steps (i) and (iii) in order to generate a new viral strain which has at least one viral genome segment from the parent virus. Using reverse genetics between steps (i) and (iii) separates the viral products used in step (i) from the viral products used in step (iii), and so can act as a filter against adventitious agents that may have been introduced before step (i). In addition to being used as a 'filter' in this way, reverse genetics techniques can also be used for other reasons *e.g*. to generate reassortants, to manipulate coding sequences, to replace specific segments, *etc.* Further details are given below in relation to the second aspect of the disclosure.

### Using reverse genetics in conjunction with cell culture of influenza viruses

A second aspect of the disclosure provides a process for preparing an influenza seed virus for vaccine manufacture, comprising steps of: (i) infecting a cell line with an influenza virus obtained either directly from a patient or from a primary isolate; (ii) preparing a cDNA of at least one viral RNA segment of an influenza virus produced by the infected cell line obtained in step (i), and using the cDNA in a reverse genetics procedure to prepare a new influenza virus having at least one viral RNA segment in common with the influenza virus of step (i); and (iii) infecting a cell line with the new influenza virus, and then culturing the cell line in order to produce the new influenza virus.

The virus used in step (i) may be an influenza A virus of any subtype, or may be an influenza B virus. Preferred influenza A virus subtypes are H1, H3 and H5.

None of steps (i), (ii) or (iii) involves growth or passaging of the virus in eggs. Preferably they all take place in the same type of cell *e.g.* they all take place in MDCK cells.

Further features of this second aspect of the disclosure are as described above for the first aspect. Thus the use of MDCK cells is preferred, *etc.*

Reverse genetics techniques are described in more detail below. The genome segment(s) transferred in step (ii) will include the HA segment, and may include the NA segment and/or one or more further segments.

### Seed viruses

The first and second aspects of the disclosure provide seed viruses. These seed viruses can be used in various ways.

Seed viruses can be characterised *e.g*. to sequence their nucleic acids and/or proteins, to check their antigenic relatedness to other strains (*e.g.* circulating strains), to check their immunogenicity, *etc.* Sequencing the viral HA gene to reveal HA amino acid sequence is typical.

Seed viruses can be used to elicit anti-sera.

Seed viruses can be distributed to vaccine manufacturers.

Seed viruses can be stored for future use.

Seed viruses can be used to prepare working seed lots. This system allows safe storage of the original seed virus while day-to-day use is performed with the working seed lots. Working seeds may be frozen until required. The preparation of a working seed lot may involve a step of viral growth in cell culture, preferably on the same cell type as used in preparation of the seed virus. Growth in eggs is not used for preparing working seed lots.

Seed viruses can be used to infect cell lines for growth to provide viruses for vaccine manufacture or for use in preparing diagnostic tests.

Seed viruses of the disclosure share many of the characteristics of current egg-derived seed viruses, but can differ in various ways.

For instance, preferred influenza A seed viruses of the disclosure include fewer than 6 (*i.e.* 0, 1, 2, 3, 4 or 5) viral segments from a PR/8/34 influenza virus, as described in more detail below.

### Egg-free vaccine production

A third aspect of the disclosure provides a process for preparing an influenza virus for vaccine manufacture, comprising steps of: (i) obtaining an influenza virus that is either circulating in the population or has a hemagglutinin that is antigenically representative of an influenza virus that is circulating in the population; (ii) infecting a cell line with the influenza virus obtained in step (i); (iii) passaging virus obtained from the infected cell line in step (ii) at least once, to give a seed strain; and (iv) culturing the seed strain from step (iii) in order to produce influenza virus.

In the same way that the first and second aspects differ from each other (see above), a fourth aspect of the disclosure provides a process for preparing an influenza virus for vaccine manufacture, comprising steps of: (i) obtaining an influenza virus that is either circulating in the population or has a hemagglutinin that is antigenically representative of an influenza virus that is circulating in the population; (ii) infecting a cell line with the influenza virus obtained in step (i); (iii) preparing a cDNA of at least one viral RNA segment of an influenza virus produced by the infected cell line obtained in step (i), and using the cDNA in a reverse genetics procedure to prepare a influenza seed virus having at least one viral RNA segment in common with the influenza virus of step (i); and (iv) infecting a cell line with the influenza seed virus, and then culturing the passaged cell line from step (iii) in order to produce influenza virus.

Also provided is a process for preparing an influenza virus vaccine, comprising these steps (i) to (iv) of the third or fourth aspect, followed by step: (v) treating virus obtained in step (iv) to give a vaccine. Details of techniques used in step (v) are given below.

None of steps (i), (ii), (iii), (iv) or (v) involves growth or passaging of the virus in eggs.

The virus used in step (i) may be an influenza A virus of any subtype, or may be an influenza B virus. Preferred influenza A virus subtypes are H1, H3 and H5 *e.g.* a H1N1 or H3N2.

The virus used in step (i) has not been propagated on eggs since its primary isolation, and preferably is not propagated on eggs at any stage between the patient who originally presented the virus and the beginning of step (i).

The term "antigenically representative" is used in the influenza vaccine art to describe viral strains which may not actually be in widespread circulation within the population, but which elicit immune responses that can protect against strains that are in circulation while being convenient for manufacturing purposes. Sera elicited by an "antigenically representative" strain will be able to inhibit a circulating strain *e.g*. in a hemagglutination inhibition assay.

Step (i) may involve starting with a number of different strains and then selecting a strain for further use. For instance, it may involve starting with a number of different H1N1 strains of influenza A virus and then selecting a strain for further use. It may involve starting with a number of different H3N2 strains of influenza A virus and then selecting a strain for further use. It may involve starting with a number of different strains of influenza B virus and then selecting a strain for further use. The selection will be based on the immunological and serological criteria that are routinely used when selecting strains for inclusion in influenza viruses *e.g*. to choose a strain that is antigenically representative of the most common and/or pathogenic strains in circulation.

Step (iii) may be followed be a step in which the seed virus is confirmed as being antigenically representative of the strain obtained in step (i). This verification step may be performed before step (iv) begins, or may be performed in parallel to step (iv).

Also provided is a process for preparing an influenza virus vaccine, comprising steps of: (a) obtaining a virus that has been prepared by a method comprising either steps (i) to (iii) of the first aspect or steps (i) to (iv) of the second aspect; and (b) treating this virus to give a vaccine.

Overall, therefore, the third and fourth aspects allow the production of influenza vaccines from a patient sample (or a primary isolate), where the influenza virus used to prepare the vaccine is not passaged through egg at any stage.

### Virus isolation

As mentioned above, there is a strong bias towards using eggs for influenza virus isolation. Instead, the invention uses MDCK cells. As explained above, the invention provides a process for manufacturing a vaccine comprising steps of: (i) isolating an influenza virus from a patient sample, comprising a step in which the patient sample is incubated with a MDCK cell, wherein the MDCK cell is growing in a serum-free suspension culture, and (ii) manufacturing a vaccine from the influenza virus isolated in step (i), wherein the influenza virus is not grown in eggs before being exposed to MDCK cells, and wherein after isolation, the virus is not subsequently grown in eggs. The invention also provides a process for preparing an influenza virus antigen for use in a vaccine, comprising steps of: (i) receiving an influenza virus isolated from a patient sample by a method comprising a step in which the patient sample is incubated with a MDCK cell, wherein the MDCK cell is growing in a serum-free suspension culture; (ii) infecting a cell line with this influenza virus; and (iii) culturing the infected cells from step (ii) in order to produce influenza virus, wherein the influenza virus is not grown in eggs before being exposed to MDCK cells, and wherein after isolation, the virus is not subsequently grown in eggs.

In some embodiments, the MDCK cells are grown in a protein-free medium. In other embodiments, the MDCK cells are non-tumorigenic. In other embodiments, the MDCK cells are not grown in the presence of an overlay medium.

In the method for isolating an influenza virus from a patient sample, comprising a step in which the patient sample is incubated with a MDCK cell, the MDCK cell may be growing in a protein-free medium, the patient sample may be incubated with a non-tumorigenic MDCK cell and/or the MDCK cell may not be provided with an overlay medium.

According to the invention the patient sample is incubated with a MDCK cell, wherein the MDCK cell is growing in a serum-free suspension culture.

The patient sample may be incubated with a MDCK cell, wherein the MDCK cell is growing in a protein-free suspension culture.

The patient sample may be incubated with a non-tumorigenic MDCK cell, wherein the MDCK cell is growing in a serum-free suspension culture.

The patient sample may be incubated with a non-tumorigenic MDCK cell, wherein the MDCK cell is growing in a protein-free suspension culture.

An influenza virus may be isolated by one of these methods. Such a virus may also be used in vaccine manufacture.

Incubation of the patient sample and the MDCK cell generally results in infection of the MDCK cell by an influenza virus, such as a human influenza virus, and in particular a human influenza A virus. The virus can replicate in the cells, and the replicated virus can then be collected. Optionally, it can be used in downstream method steps *e.g*. detection, characterisation, analysis, preparation of seed virus, manipulation, *etc.*

After isolation in MDCK cells, a virus may also be passaged and/or grown in MDCK cells.

The invention involves the use of MDCK cell lines. The original MDCK cell line is available from the ATCC, but the invention uses derivatives of this cell line. As shown below, isolation in these derivatives has been shown to be superior to isolation in the original MDCK cell line. Suitable MDCK cells and their characteristics are discussed in more detail below.

For instance, some embodiments use a MDCK cell line that can spontaneously replicate in suspension culture. Reference 30 discloses a MDCK cell line that was adapted for growth in suspension culture, and this cell line ('MDCK 33016') is particularly useful for the methods of the invention. MDCK 33016 can grow in serum-free culture, and can grow without needing an overlay medium. Another MDCK cell line that can grow in suspension culture, including in serum-free culture, is the 'B-702' cell line [36; see below].

Non-tumorigenic MDCK cell lines for use with the invention include those disclosed in reference 37, such as 'MDCK-S', 'MDCK-SF101', 'MDCK-SF102' and 'MDCK-SF103' (see below).

In some embodiments of the invention, the MDCK cells grow in serum-free culture media and/or protein free media.

Unlike certain prior art methods, the invention can avoid the need to use an overlay medium during influenza virus isolation.

It is preferred that a virus is not grown in eggs before being exposed to MDCK cells as described above. It may also be preferred that virus grown in MDCK cells, as described above, is not subsequently grown in eggs.

For the patient sample used in the invention, clinical samples used in influenza virus isolation can take various forms, but typically comprise respiratory secretions, including but not limited to: direct aspirates; gargles; nasal washings; nasal swabs; throat swabs; pharyngeal swabs; *etc.* These are generally taken from a patient suspected of having an influenza virus infection, including patients who may be harbouring a new strain of influenza virus.

Influenza viruses isolated by the methods described herein may be used to prepare an influenza seed virus for vaccine manufacture. Thus a method described herein may include a further step of passaging the virus from an infected MDCK cell line at least once. The method may then include a step of culturing the infected cells in order to produce influenza virus. Influenza virus purified after this culture step may be used as a seed virus, as described elsewhere herein.

A virus isolated according to the the methods described herein may also be used as a source for reverse genetics techniques. Thus cDNA may be prepared from at least one viral RNA segment of an influenza virus isolated according to the methods described herein. The cDNA may then be used in a reverse genetics procedure to prepare a new influenza virus having at least one viral RNA segment in common with the isolated influenza virus. This new influenza virus may then be used to infect a cell line *e.g.* for further culture.

The methods described herein may be used to isolate any suitable influenza virus, including human influenza viruses. These may be influenza A viruses, influenza B viruses or influenza C viruses. Influenza A viruses are typical, and useful influenza A virus subtypes are H1, H3 and H5.

In the current inter-pandemic period, vaccines typically include two influenza A strains (H1N1 and H3N2) and one influenza B strain. The methods described herein may be used to isolate such strains, or to isolate pandemic viral strains, such as H2, H5, H7 or H9 subtype strains. In general, the methods described herein may be used to isolate an influenza A virus having one of HA subtypes H1, H2, H3, H4, H5, H6, H7, H8, H9, H10, H11, H12, H13, H14, H15 or H16.

Influenza viruses isolated according to the invention may include hemagglutinin with a binding preference for oligosaccharides with a Sia(α2,6)Gal terminal disaccharide compared to oligosaccharides with a Sia(α2,3)Gal terminal disaccharide. Advantageously, the isolation methods of the invention have been found to assist in stable retention of a virus's HA sequence, and thus its oligosaccharide preference.

Viruses isolated according to the methods described herein may be used in vaccine manufacture and in methods of treatment. Thus an antigen prepared from a virus isolated according to the methods described herein may be used in the manufacture of a medicament for raising an immune response in a patient.

### Receptor binding

Human influenza viruses bind to receptor oligosaccharides having a Sia(α2,6)Gal terminal disaccharide (sialic acid linked α-2,6 to galactose), but eggs instead have receptor oligosaccharides with a Sia(α2,3)Gal terminal disaccharide. Growth of human influenza viruses in eggs provides selection pressure on hemagglutinin away from Sia(α2,6)Gal binding towards Sia(α2,3)Gal binding.

Like eggs, Vero cells express predominantly Sia(α2,3)Gal receptors [15]. In contrast, MDCK cells and PER.C6 cells express both Sia(α2,3)Gal and Sia(α2,6)Gal. Reference 16 reports transfection of MDCK cells to overexpress α-2,6-sialyltransferase in order to favour selection of Sia(α2,6)Gal binding. Even without such manipulations, however, it is possible to grow influenza viruses on MDCK cells without shifting them towards Sia(α2,3)Gal binding. Thus the invention can use cells that express both Sia(α2,3)Gal and Sia(α2,6)Gal, but can produce influenza viruses that have a binding preference for oligosaccharides with a Sia(α2,6)Gal terminal disaccharide compared to oligosaccharides with a Sia(α2,3)Gal terminal disaccharide.

In preferred embodiments of the first and second aspects, influenza viruses used for infection in step (i) have a binding preference for oligosaccharides with a Sia(α2,6)Gal terminal disaccharide compared to oligosaccharides with a Sia(α2,3)Gal terminal disaccharide. This binding preference is retained during step (ii) and step (iii), such that the influenza virus produced in step (iii) has a binding preference for oligosaccharides with a Sia(α2,6)Gal terminal disaccharide compared to oligosaccharides with a Sia(α2,3)Gal terminal disaccharide.

In preferred embodiments of the third and fourth aspects, influenza viruses used for infection in step (ii) have a binding preference for oligosaccharides with a Sia(α2,6)Gal terminal disaccharide compared to oligosaccharides with a Sia(α2,3)Gal terminal disaccharide. This binding preference is retained during step (iii) and step (iv), such that the influenza virus produced in step (iv) has a binding preference for oligosaccharides with a Sia(α2,6)Gal terminal disaccharide compared to oligosaccharides with a Sia(α2,3)Gal terminal disaccharide.

To determine if a virus has a binding preference for oligosaccharides with a Sia(α2,6)Gal terminal disaccharide compared to oligosaccharides with a Sia(α2,3)Gal terminal disaccharide, various assays can be used. For instance, reference 17 describes a solid-phase enzyme-linked assay for influenza virus receptor-binding activity which gives sensitive and quantitative measurements of affinity constants. Reference 18 used a solid-phase assay in which binding of viruses to two different sialylglycoproteins was assessed (ovomucoid, with Sia(α2,3)Gal determinants; and pig α₂-macroglobulin, which Sia(α2,6)Gal determinants), and also describes an assay in which the binding of virus was assessed against two receptor analogs: free sialic acid (Neu5Ac) and 3'-sialyllactose (Neu5Acα2-3Galβ1-4Glc). Reference 19 reports an assay using a glycan array which was able to clearly differentiate receptor preferences for α2,3 or α2,6 linkages. Reference 20 reports an assay based on agglutination of human erythrocytes enzymatically modified to contain either Sia(α2,6)Gal or Sia(α2,3)Gal. Depending on the type of assay, it may be performed directly with the virus itself, or can be performed indirectly with hemagglutinin purified from the virus.

### Viruses (including seed viruses) prepared or isolated by techniques of the invention

Preferred influenza A viruses (including seed viruses, viruses isolated from patient samples using MDCK cells, reassortant viruses, *etc.*) include fewer than 6 (*i.e.* 0, 1, 2, 3, 4 or 5) viral segments from a PR/8/34 influenza virus. Preferably they include no PR/8/34 segments. If any PR/8/34 segment(s) is/are present then this/these will not include the PR/8/34 HA segment and usually will not include the PR/8/34 NA segment. Thus preferred viruses are those in which at least one of segments NP, M, NS, PA, PB1 and/or PB2 is not derived from PR/8/34. More preferably, at least one of segments NP, M, PA, PB1 and/or PB2 is not derived from PR/8/34. Thus the preferred viruses can improve over existing vaccines by adding to the normal HA and NA antigens one or more further epitope-containing antigens that is/are representative of a circulating strain.

Similarly, preferred influenza A viruses include fewer than 6 (*i.e.* 0, 1, 2, 3, 4 or 5) viral segments from an AA/6/60 influenza virus (A/Ann Arbor/6/60). Preferably they include no AA/6/60 segments. If any AA/6/60 segment(s) is/are present then this/these will not include the AA/6/60 HA segment and usually will not include the AA/6/60 NA segment. Thus preferred viruses are those in which at least one of segments NP, M, NS, PA, PB1 and/or PB2 is not derived from AA/6/60. More preferably, at least one of segments NP, M, PA, PB1 and/or PB2 is not derived from AA/6/60.

Preferred influenza B viruses include fewer than 6 (*i.e.* 0, 1, 2, 3, 4 or 5) viral segments from an AA/1/66 influenza virus (B/Ann Arbor/1/66). Preferably they include no AA/1/66 segments. If any AA/1/66 segment(s) is/are present then this/these will not include the AA/1/66 HA segment and usually will not include the AA/1/66 NA segment. Thus preferred viruses are those in which at least one of segments NP, M, NS, PA, PB1 and/or PB2 is not derived from AA/1/66. More preferably, at least one of segments NP, M, PA, PB1 and/or PB2 is not derived from AA/1/66.

Preferred influenza viruses (including seed viruses, viruses isolated from patient samples using MDCK cells, reassortant viruses, *etc.*) include hemagglutinin with a binding preference for oligosaccharides with a Sia(α2,6)Gal terminal disaccharide compared to oligosaccharides with a Sia(α2,3)Gal terminal disaccharide. This binding preference is discussed in more detail above.

Preferred influenza viruses (including seed viruses, viruses isolated from patient samples using MDCK cells, reassortant viruses, *etc.*) include glycoproteins (including hemagglutinin) with a different glycosylation pattern from egg-derived viruses. Thus the glycoproteins will comprise glycoforms that are not seen in viruses grown in chicken eggs *e.g*. they may have non-avian sugar linkages, including mammalian sugar linkages.

### Cell lines

The invention involves the use of cell lines that support influenza virus replication, and avoids the use of eggs. The cell line will typically be of mammalian origin. Suitable mammalian cells of origin include, but are not limited to, hamster, cattle, primate (including humans and monkeys) and dog cells, although the use of primate cells is not preferred. Various cell types may be used, such as kidney cells, fibroblasts, retinal cells, lung cells, *etc.* Examples of suitable hamster cells are the cell lines having the names BHK21 or HKCC. Suitable monkey cells are *e.g*. African green monkey cells, such as kidney cells as in the Vero cell line [21-23]. Suitable dog cells are *e.g*. kidney cells, as in the CLDK and MDCK cell lines.

Thus suitable cell lines include, but are not limited to: MDCK; CHO; CLDK; HKCC; 293T; BHK; Vero; MRC-5; PER.C6 [24]; FRhL2; WI-38; *etc.* Suitable cell lines are widely available *e.g.* from the American Type Cell Culture (ATCC) collection [25], from the Coriell Cell Repositories [26], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. Any of these cell types can be used for growth, reassortment and/or passaging according to the invention.

The most preferred cell lines are those with mammalian-type glycosylation. As a less-preferred alternative to mammalian cell lines, virus can be grown on avian cell lines [*e.g.* refs. 27-29], including cell lines derived from ducks (*e.g.* duck retina) or hens *e.g.* chicken embryo fibroblasts (CEF), *etc.,* but the use of mammalian cells means that vaccines can be free from avian DNA and egg proteins (such as ovalbumin and ovomucoid), thereby reducing allergenicity.

The most preferred cell lines for growing influenza viruses are MDCK cell lines [30-33], derived from Madin Darby canine kidney. The original MDCK cell line is available from the ATCC as CCL-34, but derivatives of this cell line may also be used. For instance, reference 30 discloses a MDCK cell line that was adapted for growth in suspension culture ('MDCK 33016' or '33016-PF', deposited as DSM ACC 2219; see also refs. 34 & 35). Similarly, reference 36 discloses a MDCK-derived cell line that grows in suspension in serum-free culture ('B-702', deposited as FERM BP-7449). Reference 37 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (ATCC PTA-6503). Reference 38 discloses MDCK cell lines with high susceptibility to infection, including 'MDCK.5F1' cells (ATCC CRL-12042) Any of these MDCK cell lines can be used with the invention.

Virus may be grown on cells in adherent culture or in suspension. Microcarrier cultures can also be used. In some embodiments, the cells may thus be adapted for growth in suspension.

Cell lines are preferably grown in serum-free culture media and/or protein free media. In the context of the present invention a medium is referred to as a serum-free medium when it has no additives from serum of human or animal origin. The cells growing in such cultures naturally contain proteins themselves, but a protein-free medium is understood to mean one in which multiplication of the cells occurs with exclusion of (without addition to the culture medium of) proteins, growth factors, other protein additives and non-serum proteins, but can optionally include (in the culture medium) proteins such as trypsin or other proteases that may be necessary for viral growth.

Cell lines supporting influenza virus replication are preferably grown below 37°C [39] (*e.g.* 30-36°C, or at about 30°C, 31°C, 32°C, 33°C, 34°C, 35°C, 36°C) during viral replication. For instance, in the methods of the invention, MDCK cells may be grown (before, during or after the isolation step) at these temperatures, particularly during viral replication.

Methods for propagating influenza virus in cultured cells (*e.g.* for growing influenza virus in cultured MDCK cells according to the invention) generally includes the steps of inoculating a culture of cells with an inoculum of the strain to be grown, cultivating the infected cells for a desired time period for virus propagation, such as for example as determined by virus titer or antigen expression (*e.g*. between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (*e.g*. monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture e.g. before inoculation, at the same time as inoculation, or after inoculation [39].

In preferred embodiments, particularly with MDCK cells, a cell line is not passaged from a master working cell bank beyond 40 population-doubling levels.

The viral inoculum and the viral culture are preferably free from (*i.e.* will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [40]. Similarly, preferred MDCK cell lines used with the methods of the invention are free from (*i.e.* will have been tested for and given a negative result for infection by) herpes simplex viruses, respiratory syncytial viruses, parainfluenza virus 3, SARS coronavirus, adenoviruses, rhinoviruses, reoviruses, polyomaviruses, birnaviruses, circoviruses, and/or parvoviruses. Absence of herpes simplex viruses is particularly preferred.

A MDCK cell line used with the invention preferably contains no marker for G418 resistance (*cf.* reference 16). Thus the cell line may be sensitive to G418 treatment.

The cell line used with the invention preferably contains no exogenous plasmids (*cf.* reference 16), except for any that may be required for reverse genetics techniques.

### Reverse genetics techniques

As mentioned above, the invention can be used directly with clinical isolates or primary isolates. In addition, however, the invention can be used with reassortant strains, including those generated using reverse genetic techniques [*e.g.* 41-45]. Reverse genetics techniques can use *in vitro* manipulation of plasmids to generate combinations of viral segments, to facilitate manipulation of coding or non-coding sequences in the viral segments, to introduce mutations, *etc.* The techniques can be used for both influenza A and influenza B viruses.

Reverse genetics typically involves expressing (a) DNA molecules that encode desired viral RNA molecules *e.g*. from polI promoters, bacterial RNA polymerase promoters, bacteriophage polymerase promoters, *etc.* and (b) DNA molecules that encode viral proteins *e.g.* from polII promoters, such that expression of both types of DNA in a cell leads to assembly of a complete intact infectious virion.

The DNA preferably provides all of the viral RNA and proteins, but it is also possible to use a helper virus to provide some of the RNA and proteins. Plasmid-based methods using separate plasmids for producing each viral RNA are preferred [46-48], and these methods will also involve the use of plasmids to express all or some (*e.g*. just PB1, PB2, PA & NP proteins) of the viral proteins, with 12 plasmids being used in some methods.

To reduce the number of plasmids needed, a recent approach [49] combines a plurality of RNA polymerase I transcription cassettes (for viral RNA synthesis) on the same plasmid (*e.g*. sequences encoding 1, 2, 3, 4, 5, 6, 7 or all 8 influenza A vRNA segments), and a plurality of protein-coding regions with RNA polymerase II promoters on another plasmid (*e.g*. sequences encoding 1, 2, 3, 4, 5, 6, 7 or all 8 influenza A mRNA transcripts). Preferred aspects of the reference 49 method involve: (a) PB1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

Because of the species-specificity of polI promoters, the canine polI promoter [50] may be used when performing reverse genetics in MDCK cells. As an alternative to using polI promoters to encode the viral RNA segments, it is possible to use bacteriophage polymerase promoters [51]. For instance, promoters for the SP6, T3 or T7 polymerases can conveniently be used. Because of the species-specificity of polI promoters, bacteriophage polymerase promoters can be more convenient for many cell types (*e.g.* MDCK), although a cell must also be transfected with a plasmid encoding the exogenous polymerase enzyme.

In other techniques it is possible to use dual polI and polII promoters to simultaneously code for the viral RNAs and for expressible mRNAs from a single template [52,53].

Whereas strains used for growth in eggs usually include six RNA segments from a PR/8/34 influenza A virus (with HA and N segments being from a vaccine strain, *i.e.* a 6:2 reassortant), the avoidance of eggs with the invention means that PR/8/34 segments can be omitted. Influenza A viruses can thus include fewer than 6 (*i.e.* 0, 1, 2, 3, 4 or 5) viral segments from a PR/8/34 influenza virus. Thus preferred viruses are those in which at least one of segments NP, M, NS, PA, PB1 and/or PB2 is not derived from PR/8/34. A virus may include a NS segment that originated in an avian influenza virus.

Where the invention uses reverse genetics, it allows a viral RNA segment from a source influenza virus to be transferred into the genome of a destination influenza virus. These two viruses will thus have at least one viral RNA segment in common. The term "in common" here means an identical copy of the whole segment, but can also extend to mean a modified copy of the segment with modifications in the coding and/or non-coding regions. Where modifications are made in the coding-region, these will not substantially change the immunogenicity and/or activity of the encoded protein. Thus a HA segment may be manipulated around the HA1/HA2 cleavage site without changing its ability to elicit effective anti-HA antibodies when administered to a patient. Thus reverse genetics may be used to modify the natural HA of a virus isolated according to the methods of the invention *e.g.* to remove determinants (*e.g.* hyper-basic regions around the HA1/HA2 cleavage site) that cause a virus to be highly pathogenic in avian species.

### Vaccine preparation

Various forms of influenza virus vaccine are currently available (*e.g*. see chapters 17 & 18 of reference 54). Vaccines are generally based either on live virus or on inactivated virus. Inactivated vaccines may be based on whole virions, 'split' virions, or on purified surface antigens. Influenza antigens can also be presented in the form of virosomes. The invention can be used when manufacturing vaccines based on inactivated virus.

Live viruses include MedImmune's FLUMIST^{™} product (trivalent live virus). Vaccine is prepared by a process that comprises growing the virus on a suitable substrate and then purifying virions from virion-containing fluids. For example, the fluids may be clarified by centrifugation, and stabilized with buffer (*e.g*. containing sucrose, potassium phosphate, and monosodium glutamate).

Where an inactivated virus is used, the vaccine may comprise whole virion, split virion, or purified surface antigens (including hemagglutinin and, usually, also including neuraminidase). Chemical means for inactivating a virus include treatment with an effective amount of one or more of the following agents: detergents, formaldehyde, β-propiolactone, methylene blue, psoralen, carboxyfullerene (C60), binary ethylamine, acetyl ethyleneimine, or combinations thereof. Non-chemical methods of viral inactivation are known in the art, such as for example UV light or gamma irradiation.

Virions can be harvested from virus-containing fluids by various methods. For example, a purification process may involve zonal centrifugation using a linear sucrose gradient solution that includes detergent to disrupt the virions. Antigens may then be purified, after optional dilution, by diafiltration.

Split virions are obtained by treating purified virions with detergents (*e.g*. ethyl ether, polysorbate 80, deoxycholate, tri-*N*-butyl phosphate, Triton X-100, Triton N101, cetyltrimethylammonium bromide, Tergitol NP9, *etc*.) to produce subvirion preparations, including the 'Tween-ether' splitting process. Methods of splitting influenza viruses are well known in the art *e.g.* see refs. 55-60, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (*e.g.* cationic) surfactants *e.g.* alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, NP9, quaternary ammonium compounds, sarcosyl, CTABs (cetyl trimethyl ammonium bromides), tri-*N*-butyl phosphate, Cetavlon, myristyltrimethylammonium salts, lipofectin, lipofectamine, and DOT-MA, the octyl- or nonylphenoxy polyoxyethanols (*e.g*. the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde, and splitting can take place during initial virion purification (e.g. in a sucrose density gradient solution). Thus a splitting process can involve clarification of the virion-containing material (to remove non-virion material), concentration of the harvested virions (*e.g*. using an adsorption method, such as CaHPO₄ adsorption), separation of whole virions from non-virion material, splitting of virions using a splitting agent in a density gradient centrifugation step (*e.g*. using a sucrose gradient that contains a splitting agent such as sodium deoxycholate), and then filtration (*e.g*. ultrafiltration) to remove undesired materials. Split virions can usefully be resuspended in sodium phosphate-buffered isotonic sodium chloride solution. The BEGRIVAC^{™}, FLUARIX^{™}, FLUZONE^{™} and FLUSHIELD^{™} products are split vaccines.

Purified surface antigen vaccines comprise the influenza surface antigens hemagglutinin and, typically, also neuraminidase. Processes for preparing these proteins in purified form are well known in the art. The FLUVIRIN^{™}, AGRIPPAL^{™} and INFLUVAC^{™} products are subunit vaccines.

Another form of inactivated influenza antigen is the virosome [61] (nucleic acid free viral-like liposomal particles). Virosomes can be prepared by solubilization of influenza virus with a detergent followed by removal of the nucleocapsid and reconstitution of the membrane containing the viral glycoproteins. An alternative method for preparing virosomes involves adding viral membraneglycoproteins to excess amounts of phospholipids, to give liposomes with viral proteins in their membrane. The invention can be used to store bulk virosomes. as in the INFLEXAL V^{™} and INVAVAC^{™} products.

The influenza virus may be attenuated. The influenza virus may be temperature-sensitive. The influenza virus may be cold-adapted. These three features are particularly useful when using live virus as an antigen.

HA is the main immunogen in current inactivated influenza vaccines, and vaccine doses are standardised by reference to HA levels, typically measured by SRID. Existing vaccines typically contain about 15µg of HA per strain, although lower doses can be used *e.g.* for children, or in pandemic situations, or when using an adjuvant. Fractional doses such as ½ (*i.e.* 7.5µg HA per strain), ¼ and ⅛ have been used [81,82], as have higher doses (*e.g.* 3x or 9x doses [62,63]). Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc.* Particular doses include *e.g.* about 45, about 30, about 15, about 10, about 7.5, about 5, about 3.8, about 1.9, about 1.5, *etc.* per strain.

For live vaccines, dosing is measured by median tissue culture infectious dose (TCID₅₀) rather than HA content, and a TCID₅₀ of between 10⁶ and 10⁸ (preferably between 10^{6.5}-10^{7.5}) per strain is typical.

Strains used with the invention may have a natural HA as found in a wild-type virus, or a modified HA. For instance, it is known to modify HA to remove determinants (*e.g*. hyper-basic regions around the HA1/HA2 cleavage site) that cause a virus to be highly pathogenic in avian species.

Influenza virus strains for use in vaccines change from season to season. In the current inter-pandemic period, vaccines typically include two influenza A strains (H1N1 and H3N2) and one influenza B strain, and trivalent vaccines are typical. The invention may also use pandemic viral strains (*i.e*. strains to which the vaccine recipient and the general human population are immunologically naive, in particular of influenza A virus), such as H2, H5, H7 or H9 subtype strains, and influenza vaccines for pandemic strains may be monovalent or may be based on a normal trivalent vaccine supplemented by a pandemic strain. Depending on the season and on the nature of the antigen included in the vaccine, however, the invention may protect against one or more of HA subtypes H1, H2, H3, H4, H5, H6, H7, H8, H9, H10, H11, H12, H13, H14, H15 or H16. The invention may protect against one or more of influenza A virus NA subtypes N1, N2, N3, N4, N5, N6, N7, N8 or N9.

As well as being suitable for immunizing against inter-pandemic strains, the compositions that may be produced by the invention are particularly useful for immunizing against pandemic strains. The characteristics of an influenza strain that give it the potential to cause a pandemic outbreak are: (a) it contains a new hemagglutinin compared to the hemagglutinins in currently-circulating human strains, *i.e.* one that has not been evident in the human population for over a decade (*e.g.* H2), or has not previously been seen at all in the human population (*e.g*. H5, H6 or H9, that have generally been found only in bird populations), such that the human population will be immunologically naive to the strain's hemagglutinin; (b) it is capable of being transmitted horizontally in the human population; and (c) it is pathogenic to humans. A virus with H5 hemagglutinin type is preferred for immunizing against pandemic influenza, such as a H5N1 strain. Other possible strains include H5N3, H9N2, H2N2, H7N1 and H7N7, and any other emerging potentially pandemic strains. Within the H5 subtype, a virus may fall into HA clade 1, HA clade 1', HA clade 2 or HA clade 3 [64], with clades 1 and 3 being particularly relevant.

Other strains whose antigens can usefully be included in the compositions are strains which are resistant to antiviral therapy (*e.g*. resistant to oseltamivir [65] and/or zanamivir), including resistant pandemic strains [66].

Compositions that may be produced by the invention may thus include antigen(s) from one or more (*e.g.* 1, 2, 3, 4 or more) influenza virus strains, including influenza A virus and/or influenza B virus. Where a vaccine includes more than one strain of influenza, the different strains are typically grown separately and are mixed after the viruses have been harvested and antigens have been prepared. Thus a process of the invention may include the step of mixing antigens from more than one influenza strain. A trivalent vaccine is preferred, including antigens from two influenza A virus strains and one influenza B virus strain.

In some embodiments, the compositions may include antigen from a single influenza A strain. In some embodiments, the compositions may include antigen from two influenza A strains, provided that these two strains are not H1N1 and H3N2. In some embodiments, the compositions may include antigen from more than two influenza A strains.

The invention provides a process for preparing an influenza virus antigen for use in a vaccine, comprising steps of: (i) receiving an influenza virus isolated from a patient sample by a method comprising a step in which the patient sample is incubated with a MDCK cell, wherein the MDCK cell is growing in a serum-free suspension culture; (ii) infecting a cell line with this influenza virus; and (iii) culturing the infected cells from step (ii) in order to produce influenza virus, wherein the influenza virus is not grown in eggs before being exposed to MDCK cells, and wherein after isolation, the virus is not subsequently grown in eggs. Virus obtained in step (iii) can be used to prepare vaccines *e.g.* by methods involving inactivation, formulation, *etc.* The influenza virus received in step (i) will have one or more of the following characteristics: (a) it has never been propagated on an egg substrate; (b) it was isolated in a MDCK cell, such as a MDCK 33016 cell and/or a MDCK cell growing in serum-free medium; (c) it has never been propagated on a substrate growing in a serum-containing medium; (d) it was generated using reverse genetics techniques; (e) it is an influenza A virus with fewer than 6 viral segments from a PR/8/34 influenza virus and/or fewer than 6 viral segments from an AA/6/60 influenza virus *or* it is an influenza B virus with fewer than 6 viral segments from an AA/1/66 influenza virus; (f) it includes hemagglutinin with a binding preference for oligosaccharides with a Sia(α2,6)Gal terminal disaccharide compared to oligosaccharides with a Sia(α2,3)Gal terminal disaccharide; and/or (g) it has glycoproteins (including hemagglutinin) with a different glycosylation pattern from egg-derived viruses. Thus the influenza virus received in step (i) may have been obtained as described elsewhere herein.

### Host cell DNA

Where virus has been grown on a cell line then it is standard practice to minimize the amount of residual cell line DNA in the final vaccine, in order to minimize any oncogenic activity of the DNA.

Thus a vaccine composition prepared according to the invention preferably contains less than 10ng (preferably less than 1ng, and more preferably less than 100pg) of residual host cell DNA per dose, although trace amounts of host cell DNA may be present.

Vaccines containing <10ng (*e.g.* <1ng, <100pg) host cell DNA per 15µg of hemagglutinin are preferred, as are vaccines containing <10ng (*e.g.* <1ng, <100pg) host cell DNA per 0.25ml volume. Vaccines containing <10ng (*e.g.* <1ng, <100pg) host cell DNA per 50µg of hemagglutinin are more preferred, as are vaccines containing <10ng (*e.g.* <1ng, <100pg) host cell DNA per 0.5ml volume.

It is preferred that the average length of any residual host cell DNA is less than 500bp *e.g.* less than 400bp, less than 300bp, less than 200bp, less than 100bp, *etc.*

Contaminating DNA can be removed during vaccine preparation using standard purification procedures *e.g.* chromatography, *etc.* Removal of residual host cell DNA can be enhanced by nuclease treatment *e.g*. by using a DNase. A convenient method for reducing host cell DNA contamination is disclosed in references 67 & 68, involving a two-step treatment, first using a DNase (*e.g.* Benzonase), which may be used during viral growth, and then a cationic detergent (*e.g.* CTAB), which may be used during virion disruption. Treatment with an alkylating agent, such as β-propiolactone, can also be used to remove host cell DNA, and advantageously may also be used to inactivate virions [69].

Measurement of residual host cell DNA is now a routine regulatory requirement for biologicals and is within the normal capabilities of the skilled person. The assay used to measure DNA will typically be a validated assay [70,71]. The performance characteristics of a validated assay can be described in mathematical and quantifiable terms, and its possible sources of error will have been identified. The assay will generally have been tested for characteristics such as accuracy, precision, specificity. Once an assay has been calibrated (e.g. against known standard quantities of host cell DNA) and tested then quantitative DNA measurements can be routinely performed. Three main techniques for DNA quantification can be used: hybridization methods, such as Southern blots or slot blots [72]; immunoassay methods, such as the Threshold^{™} System [73]; and quantitative PCR [74]. These methods are all familiar to the skilled person, although the precise characteristics of each method may depend on the host cell in question *e.g*. the choice of probes for hybridization, the choice of primers and/or probes for amplification, *etc.* The Threshold^{™} system from *Molecular Devices* is a quantitative assay for picogram levels of total DNA, and has been used for monitoring levels of contaminating DNA in biopharmaceuticals [73]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA *e.g*. AppTec^{™} Laboratory Services, BioReliance^{™}, Althea Technologies, *etc.* A comparison of a chemiluminescent hybridisation assay and the total DNA Threshold^{™} system for measuring host cell DNA contamination of a human viral vaccine can be found in reference 75.

### Pharmaceutical compositions

Vaccine compositions manufactured according to the invention are pharmaceutically acceptable. They usually include components in addition to influenza antigens *e.g*. they typically include one or more pharmaceutical carrier(s) and/or excipient(s). Adjuvants may also be included. A thorough discussion of such components is available in reference 76.

Vaccine compositions will generally be in aqueous form.

Vaccine compositions may include preservatives such as thiomersal or 2-phenoxyethanol. It is preferred, however, that the vaccine should be substantially free from (*i.e.* less than 5µg/ml) mercurial material *e.g*. thiomersal-free [59,77]. Vaccines containing no mercury are more preferred. α-tocopherol succinate can be included as an alternative to mercurial compounds [59]. Preservative-free vaccines are particularly preferred.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Vaccine compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [78], but keeping osmolality in this range is nevertheless preferred.

Vaccine compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The pH of a vaccine composition will generally be between 5.0 and 8.1, and more typically between 6.0 and 8.0 *e.g.* 6.5 and 7.5, or between 7.0 and 7.8. A process of the invention may therefore include a step of adjusting the pH of the bulk vaccine prior to packaging.

The vaccine composition is preferably sterile. The vaccine composition is preferably non-pyrogenic *e.g*. containing <1 EU (endotoxin unit, a standard measure) per dose, and preferably <0.1 EU per dose. The vaccine composition is preferably gluten-free.

Vaccine compositions may include detergent *e.g*. a polyoxyethylene sorbitan ester surfactant (known as 'Tweens'), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts. Thus the vaccine may included less than 1mg/ml of each of octoxynol-10 and polysorbate 80. Other residual components in trace amounts could be antibiotics (*e.g*. neomycin, kanamycin, polymyxin B).

A vaccine composition may include material for a single immunisation, or may include material for multiple immunisations (*i.e*. a 'multidose' kit). The inclusion of a preservative is preferred in multidose arrangements. As an alternative (or in addition) to including a preservative in multidose compositions, the compositions may be contained in a container having an aseptic adaptor for removal of material.

Influenza vaccines are typically administered in a dosage volume of about 0.5ml, although a half dose (*i.e.* about 0.25ml) may be administered to children.

Compositions and kits are preferably stored at between 2°C and 8°C. They should not be frozen. They should ideally be kept out of direct light.

### Packaging of vaccine compositions

Suitable containers for compositions that may be produced according to the invention (or kit components) include vials, syringes (*e.g.* disposable syringes), nasal sprays, *etc.* These containers should be sterile.

Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a 'multidose' vial) *e.g.* 10 doses. Preferred vials are made of colorless glass.

A vial can have a cap (*e.g.* a Luer lock) adapted such that a pre-filled syringe can be inserted into the cap, the contents of the syringe can be expelled into the vial (*e.g.* to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Preferred syringes are those marketed under the trade name "Tip-Lok"^{™}.

Containers may be marked to show a half-dose volume *e.g*. to facilitate delivery to children. For instance, a syringe containing a 0.5ml dose may have a mark showing a 0.25ml volume.

Where a glass container (*e.g*. a syringe or a vial) is used, then it is preferred to use a container made from a borosilicate glass rather than from a soda lime glass.

A kit or composition may be packaged (*e.g*. in the same box) with a leaflet including details of the vaccine *e.g.* instructions for administration, details of the antigens within the vaccine, *etc.* The instructions may also contain warnings *e.g*. to keep a solution of adrenaline readily available in case of anaphylactic reaction following vaccination, *etc.*

### Methods of treatment, and administration of the vaccine

A vaccine may be manufactured according to the invention.

Vaccine compositions manufactured according to the invention are suitable for administration to human patients, and may be used in a method of raising an immune response in a patient, comprising the step of administering the composition to the patient.

A composition described herein may be for use as a medicament.

Also described herein is the use of an influenza virus antigen prepared according to the methods described herein, in the manufacture of a medicament for raising an immune response in a patient.

The immune response raised by these methods and uses will generally include an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [185]. Antibody responses are typically measured by hemagglutination inhibition, by microneutralisation, by single radial immunodiffusion (SRID), and/or by single radial hemolysis (SRH). These assay techniques are well known in the art.

Compositions can be administered in various ways. The most preferred immunisation route is by intramuscular injection (*e.g*. into the arm or leg), but other available routes include subcutaneous injection, intranasal [186-188], oral [189], intradermal [190,191], transcutaneous, transdermal [192], *etc.*

Vaccines prepared according to the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly (*e.g.* ≥50 years old, ≥60 years old, and preferably ≥65 years), the young (*e.g*. ≤5 years old), hospitalised patients, healthcare workers, armed service and military personnel, pregnant women, the chronically ill, immunodeficient patients, patients who have taken an antiviral compound (*e.g*. an oseltamivir or zanamivir compound; see below) in the 7 days prior to receiving the vaccine, people with egg allergies and people travelling abroad. The vaccines are not suitable solely for these groups, however, and may be used more generally in a population. For pandemic strains, administration to all age groups is preferred.

Preferred compositions satisfy 1, 2 or 3 of the CPMP criteria for efficacy. In adults (18-60 years), these criteria are: (1) ≥70% seroprotection; (2) ≥40% seroconversion; and/or (3) a GMT increase of 22.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g*. a parenteral prime and mucosal boost, a mucosal prime and parenteral boost, *etc.* Administration of more than one dose (typically two doses) is particularly useful in immunologically naive patients *e.g*. for people who have never received an influenza vaccine before, or for vaccinating against a new HA subtype (as in a pandemic outbreak). Multiple doses will typically be administered at least 1 week apart (*e.g*. about 2 weeks, about 3 weeks, about 4 weeks, about 6 weeks, about 8 weeks, about 10 weeks, about 12 weeks, about 16 weeks, *etc*.).

Vaccines produced by the invention may be administered to patients at substantially the same time as (*e.g*. during the same medical consultation or visit to a healthcare professional or vaccination centre) other vaccines *e.g*. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, the vaccines may be administered to patients at substantially the same time as (*e.g*. during the same medical consultation or visit to a healthcare professional) an antiviral compound, and in particular an antiviral compound active against influenza virus (e.g. oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof (*e.g*. the ethyl esters) and salts thereof (*e.g*. the phosphate salts). A preferred antiviral is (3R,4R,5S)-4-acetylamino-5-amino-3(1-ethylpropoxy)-1-cyclohexene-1-carboxylic acid, ethyl ester, phosphate (1:1), also known as oseltamivir phosphate (TAMIFLU^{™}).

### General

The term "comprising" encompasses "including" as well as "consisting" *e.g*. a composition "comprising" X may consist exclusively of X or may include something additional *e.g*. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

### BRIEF DESCRIPTION OF THE DRAWINGS

Figure 1 shows the scheme of isolation of influenza virus from clinical specimens.
Figure 2 compares HA titers of 9 viral samples isolated in MDCK-33016 cells. In each sample, the left-hand bar is at passage 2 and the right-hand bar is at passage 5.
Figure 3 compared HA titers of 10 samples of influenza viruses grown in three different MDCK cell types. For each sample, the three bars are: left, 33016 in suspension; middle, adherent 33016; and right, CCL-34 MDCK cells.
Figure 4 shows the binding of three viruses to SNA or MAA lectins. Figure 4A shows binding of an original isolate, 4B shows binding after growth in MDCK 33016 cells, and 4C shows binding after growth in eggs.

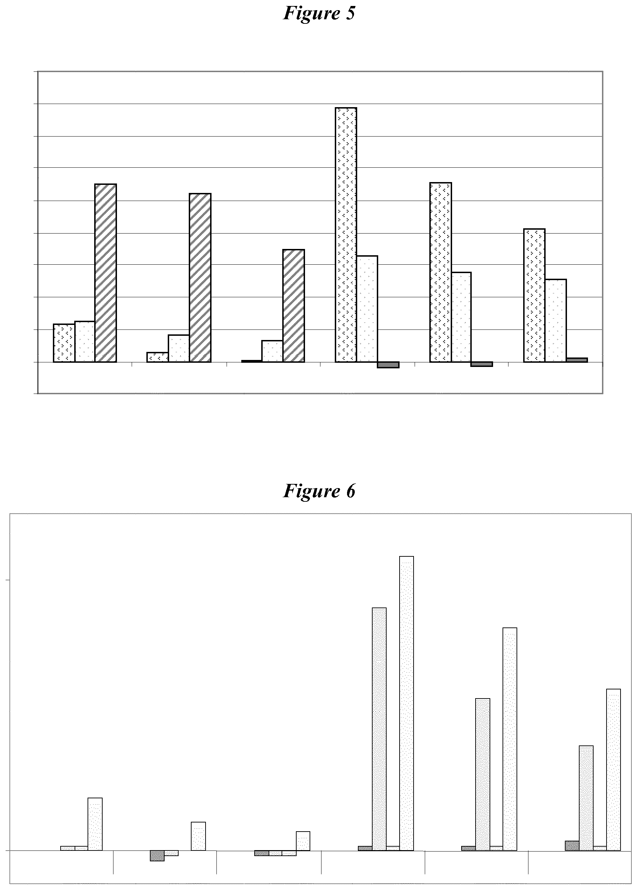

Figures 5 and 6 show the binding of viruses to 3-SL or 6-SLN. In both figures there are six groups of columns: the left-most three show binding to 3-SL at different concentrations (1µM, 0.5µM, 0.25µM) and the right-most three show binding to 6-SLN at different concentrations (0.25µM, 0.125µM, 0.0625µM). Within each of the six groups, each column shows a different virus. In Figure 5, the three columns are, from left to right: (i) a cell-isolated virus; (ii) an egg-isolated virus; and (iii) an avian virus. In Figure 6, the four columns are, from left to right: (i) virus after two passages in eggs; (ii) virus after two passages in MDCK; (iii) virus after five passages in eggs; (iv) virus after five passages in MDCK.

### MODES FOR CARRYING OUT THE INVENTION

### Virus isolation from patient samples

Clinical specimens (nasal or throat swabs) containing influenza A and/or B virus subtypes were obtained from children and adults during the 2006-2007 northern hemisphere influenza season. The susceptibility and reliability of the MDCK 33016 cell-line (DSM ACC 2219) grown in serum-free suspension culture was compared with the established MDCK CCL 34 cell-line (ATCC) and with chicken eggs, by determination of hemagglutinin (HA) titers, polymerase chain reaction (PCR), and virus titration.

248 influenza positive samples were identified by diagnostic polymerase chain reaction (PCR). Susceptibility and reliability of influenza virus replication and isolation was assessed in the MDCK 33016 cell-line and in chicken eggs by: (i) hemagglutinin (HA) titers; (ii) real-time polymerase chain reaction (PCR) for viral load measurement; and (iii) virus titration. Replication accuracy in the cells was assessed by sequencing the HA gene in the original clinical specimens and also in isolates from the second passage in MDCK cells and the chicken eggs. Virus titers obtained from isolates grown in suspension MDCK 33016 cells were compared to those from MDCK 33016 cells adhered on plates.

Results indicated that the isolation capacity of the MDCK 33016 suspension cell-line is superior to the established MDCK CCL 34 cell-line, and much greater than that of chicken eggs. After passage of virus samples in MDCK 33016 cells, amino acid substitutions were identified in no isolates. In contrast, nearly all egg-passaged viruses contained one or more amino acid substitutions, predominately in the HA1 gene. Mutations in the antibody binding site of the HA gene, observed following passage in eggs, may result in modifications to the antigenicity of the influenza virus.

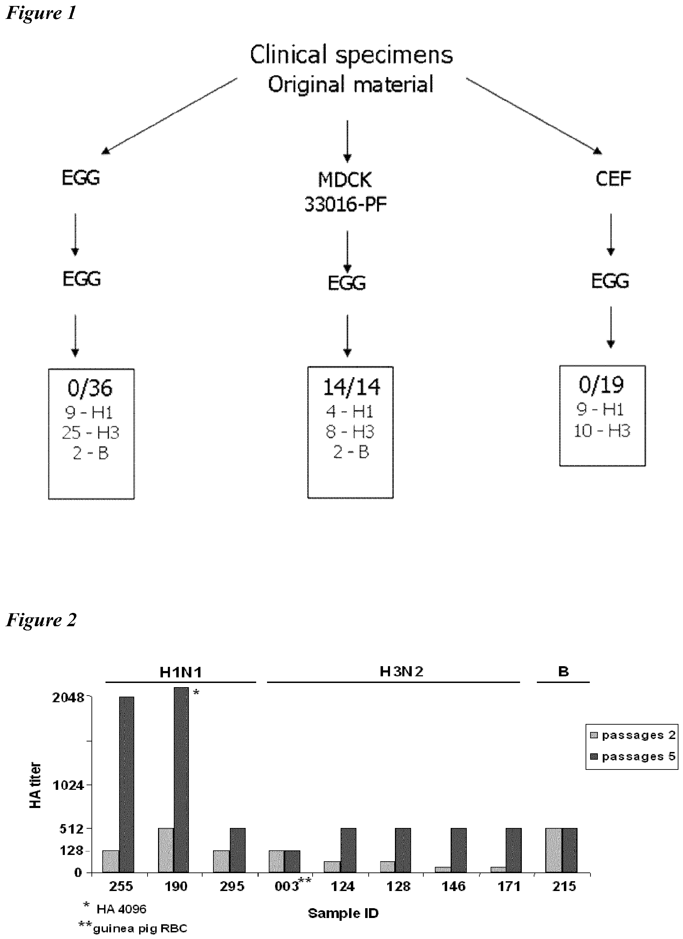

55% of clinical samples obtained from patients with acute respiratory disease were identified as influenza positive, with the following viral types: 79% A/H3N2; 12.5% A/H1N1; 1.6% B, 0.4% H3/B and 6.5% untypeable. Viral isolation from clinical specimens was possible using MDCK 33016 cells (Figure 1). In contrast, of the viruses injected into eggs from clinical specimens, none was successfully isolated. Similar negative results were achieved with freshly prepared chicken embryo fibroblasts (CEF). Isolation and establishment of influenza virus in eggs could only be achieved using supernatants of MDCK 33016 cultures with a positive HA titer.

The first harvest from each cell was further inoculated into eggs, for reference purposes. The number of successful virus isolations, using each approach, is shown in the boxes in Figure 1, with the number of different viral types injected also shown. All three virus subtypes isolated from the MDCK 33016 cells, gained reasonable HA (>32) and virus titer (>1×10⁶) after the second passage in eggs, with both titers increasing with further passages (Figure 2).

MDCK 33016 cells growing in suspension were superior to the adherent cell line (CCL-34) for isolation of influenza virus from clinical swabs for all three subtypes. The suspension cell line showed a higher sensitivity for positive influenza swab material as demonstrated by the recovery rate (Table 1). HA sequences were compared between different passages in MDCK 33016 cells and eggs to the original isolate (Table 2), and no mutations were found for influenza A strains isolated in MDCK 33016 cells even after 5 passages, whereas influenza A strains isolated in eggs showed mutations in the antibody binding site of the HA protein after 2 passages. No mutations were found for influenza B strains isolated in MDCK 33016 cells or eggs.

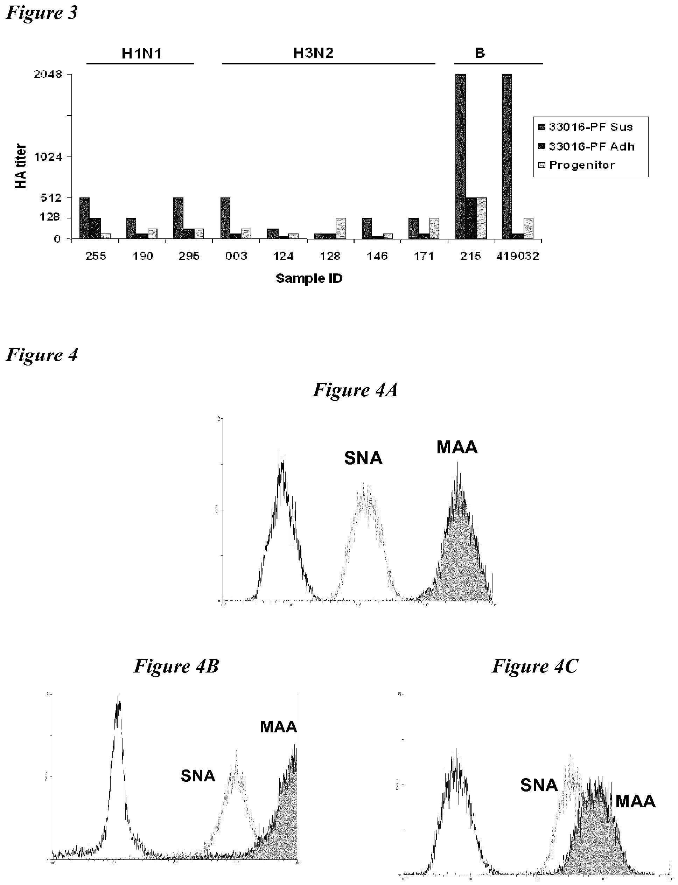

Higher virus yields, of at least one log level, were found following replication of isolates in suspension MDCK 33016 cells compared with adhered MDCK 33016 cells (Figure 3).

Thus the MDCK 33016 suspension cell-line is an ideal system for the isolation and replication of wild-type influenza strains, as it offers a greater isolation capacity compared with chicken eggs. Furthermore, due to the high replication accuracy, the use of cell-based isolates for the production of a human influenza vaccine may lead to a more authentic vaccine. Improved match between the circulating wild-type strains and those contained in the vaccine should offer greater protection against influenza for the vaccinee.

In conclusion: (a) all virus strains were successfully isolated in MDCK 33016 cells compared to eggs; (b) virus strains isolated from MDCK 33016 cells could be propagated successfully in eggs; (c) the recovery rate of all three influenza virus subtypes is superior in MDCK 33016 cells grown in suspension, when compared to the adherent cells; and (d) substitutions of the HA gene, when compared to the original material, were not present in any of the isolates grown in MDCK 33016 cells but were present after the second passage in eggs. Thus the MDCK 33016 suspension cell-line is a very suitable substrate for isolation and propagation of human influenza virus subtypes as it is highly reliable for passaging wild type influenza virus from clinical isolates and is preserves an authentic character of the wild type virus.

### Receptor binding

The receptor preferences of original isolated viruses, of egg-grown viruses and of MDCK-grown viruses were investigated. Studies used lectins with 2,3-sialyl linkages (MAA) or 2,6-sialyl linkages (SNA), or 2,3-sialyllactose (3-SL, an analog of the egg receptor) and 2,6-sialyl-N-acetyllactosamine (6-SLN, an analog of the human receptor) sialylglycopolymers [193].

Figure 4 shows the results of a representative study. The labelled peaks show binding to the SNA or MAA lectins. The original virus (4A) and the virus grown on MDCK 33016 (4B) have distinct peaks for SNA & MAA, whereas the SNA & MAA peaks substantially overlap for egg-grown virus (4C).

Binding specificity was also examined in further experiments using 3-SL and 6-SLN. An example result is shown in Figure 5. Binding on the left of the graph indicates an avian receptor preference, whereas binding on the right indicates a human receptor preference. As visible in Figure 5, the cell-isolated virus strongly favours human receptors.

Figure 6 shows data using a Stuttgart isolate (A/H1N1) after 2 or 5 passages in eggs or in MDCK 33016 grown in suspension. The MDCK-passaged viruses show a strong preference for 6-SLN.

In conclusion, all MDCK-grown clinical human A and B viruses bind to 6-SLN rather than to 3-SL, except that some isolates were found that bind to neither in this assay. Unlike the original clinical isolates, egg-adapted viruses bind either to 3-SL or to neither 3-SL nor 6-SLN.

### Changes due to growth in eggs

Various strains of influenza A and B virus were isolated in MDCK cells and then passaged up to five times through one of the following substrates: eggs; MDCK cells CCL-34; MDCK cells 33016; Vero cells; or HEK 293-T cells. The HA gene of the viruses were sequenced after each passage and HA titres were measured.

Although the HA sequence for some strains (*e.g*. A/H1N1/Bayern/7/95) was stable during passage through eggs and through MDCK 33016, for others it was not. For instance, the HA sequence of A/H1N1/Nordrhein Westfalen/1/05 acquired a mutation D203N at antibody binding site D after 2 passages through eggs, and after 2 more passages it additionally acquired a R329K. In contrast, the sequence was unaltered in viruses passaged in parallel through MDCK 33016.

For this AH1N1/NRW/1/05 strain, growth was not seen when cultured with Vero cells The other four substrates could support its growth, but the HA titres varied. For instance, titres of 32-256 were seen in eggs, but 293-T cells gave lower titres (16-32) and MDCK 33016 gave higher titres (32-512).

**TABLE 1: Recovery rate after the first passage of influenza positive samples in MDCK 33016 and ATCC (CCL-34) cell lines**

| | **Total n=248* (%)** | **Recovery rate n (%) according to viral strain** | | | |
|---|---|---|---|---|---|
| | | **A/H1N1 (n=31)** | **A/H3N2 (n=196)** | **B (n=4)** | **Untypeable (n=16)** |
| 33016 | 178 (72) | 26 (83.9) | 150 (76.5) | 4 (100) | 9 (56.3) |
| CCL-34 | 156 (63) | 23 (74.2) | 135 (68.9) | 2 (50) | 4 (25.0) |

| | | | | | |
|---|---|---|---|---|---|
| * 1 double infected (H3/B) which could be isolated in both MDCK cell lines | | | | | |

**TABLE 2: Comparison of hemagglutinin sequences after 2 or 5 passages in MDCK 33016-PF cells or eggs to the original isolate**

| **Isolate (serotype)** | **Passage (host)** | **Comparison to the original material (nucleotide)** | **Comparison to the original material (amino acid)** |
|---|---|---|---|
| 295 (H1N1) | P2 (MDCK) | 0* | 0* |
| | P2 (egg) | 1* | D203N* |
| 124 (H3N2) | P2 (MDCK) | | 0 |
| | P5 (MDCK) | 0 | 0 |
| | P2 (egg) | 1 | L210P |
| 128 (H3N2) | P2 (MDCK) | 0 | 0 |
| | P5 (MDCK) | 0 0 | 0 |
| | P2 (egg) | 3 | L210P |
| 146 (H3N2) | P2 (MDCK) | 0 | 0 |
| | P5 (MDCK) | 0 | 0 |
| | P2 (egg) | 1 | L210P |
| 171 (H3N2) | P2 (MDCK) | 0 | 0 |
| | P5 (MDCK) | 0 | 0 |
| | P2 (egg) | 1 | H199L |
| 215 (B) | P5 (MDCK) | 0** | 0** |
| | P2 (egg) | 0** | 0** |
| 419032 (B) | P2 (MDCK) | 0 | 0 |
| | P2 (egg) | 0 | 0 |

| | | | |
|---|---|---|---|
| 0 = no mutation detectable * for original isolate only the HA1 sequence was available ** comparison to the P2 (MDCK 33016) isolate | | | |

### REFERENCES

[1] Gerdil (2003) Vaccine 21:1776-9.
[2] Palese (2006) Emerging Infectious Diseases 12:61-65*.*
[3] de Oña et al. (1995) J Clin Microbiol 33:1948-49.
[4] Monto et al. (1981) J Clin Microbiol 13(1): 233-235.
[5] Govorkova et al. (1995) J Infect Dis. 172(1):250-3.
[6] Tobita et al. (1975) Med Microbiol Immunol (Berl). 162(1):9-14 and 23-27.
[7] Ollier et al. (2004) J Clin Microbiol 42(12):5861-5.
[8] Schepetiuk & Kok (1993) J Virol Methods 42(2-3):241-50.
[9] WHO (2003) Weekly epidemiological record 78:73-80
[10] US patent 6,344,354. See also WO97/38094.
[11] US patent 5,162,112
[12] Medema et al. (2004) Virus Res. 103(1-2):9-15.
[13] Robertson et al. (1995) Vaccine 13:1583-8.
[14] Merten et al. (1996) Advances in Experimental Biology 397:141-151.
[15] Govorkova et al. (1996) J. Virol., 70:5519-24.
[16] Mastrosovich et al. (2003) J Virol 77:8418-25.
[17] Gambaryan & Matrosovich (1992) J Virol Methods 39(1-2):111-23.
[18] Mastrosovich et al. (1999) J Virol 73: 1146-55.
[19] Stevens et al. (2006) J Mol Biol 355:1143-55.
[20] Couceiro & Baum (1994) Mem Inst Oswaldo Cruz 89(4):587-91.
[21] Kistner et al. (1998) Vaccine 16:960-8.
[22] Kistner et al. (1999) Dev Biol Stand 98:101-110.
[23] Bruhl et al. (2000) Vaccine 19:1149-58.
[24] Pau et al. (2001) Vaccine 19:2716-21.
*[25] http:*//*www.atcc.org*/
*[26] http:*//*locus.umdnj.edu*/
[27] WO03/076601.
[28] WO2005/042728.
[29] WO03/043415.
[30] WO97/37000.
[31] Brands et al. (1999) Dev Biol Stand 98:93-100.
[32] Halperin et al. (2002) Vaccine 20:1240-7.
[33] Tree et al. (2001) Vaccine 19:3444-50.
[34] WO03/023021
[35] WO03/023025
[36] EP-A-1260581 (WO01/64846).
[37] WO2006/071563.
[38] WO2005/113758.
[39] WO97/37001.
[40] WO2006/027698.
[41] Hoffmann et al. (2002) Vaccine 20:3165-3170.
[42] Subbarao et al. (2003) Virology 305:192-200.
[43] Liu et al. (2003) Virology 314:580-590.
[44] Ozaki et al. (2004) J. Virol. 78:1851-1857.
[45] Webby et al. (2004) Lancet 363:1099-1103.
[46] WO00/60050.
[47] WO01/04333.
[48] US patent 6649372.
[49] Neumann et al. (2005) Proc Natl Acad Sci USA 102:16825-9.
[50] WO2007/002008.
[51] WO2006/067211.
[52] WO01/83794.
[53] Hoffmann et al. (2000) Virology 267(2):310-7.
[54] Vaccines. (eds. Plotkin & Orenstein). 4th edition, 2004, ISBN: 0-7216-9688-0.
[55] WO02/28422.
[56] WO02/067983.
[57] WO02/074336.
[58] WO01/21151.
[59] WO02/097072.
[60] WO2005/113756.
[61] Huckriede et al. (2003) Methods Enzymol 373:74-91.
[62] Treanor et al. (1996) J Infect Dis 173:1467-70.
[63] Keitel et al. (1996) Clin Diagn Lab Immunol 3:507-10.
[64] World Health Organisation (2005) Emerging Infectious Diseases 11(10):1515-21.
[65] Herlocher et al. (2004) J Infect Dis 190(9):1627-30.
[66] Le et al. (2005) Nature 437(7062):1108.
[67] EP-B-0870508.
[68] US 5948410.
[69] WO2007/052163.
[70] Lundblad (2001) Biotechnology and Applied Biochemistry 34:195-197.
[71] Guidance for Industry: Bioanalytical Method Validation. U.S. Department of Health and Human Services Food and Drug Administration Center for Drug Evaluation and Research (CDER) Center for Veterinary Medicine (CVM). May 2001.
[72] Ji et al. (2002) Biotechniques. 32:1162-7.
[73] Briggs (1991) J Parenter Sci Technol. 45:7-12.
[74] Lahijani et al. (1998) Hum Gene Ther. 9:1173-80.
[75] Lokteff et al. (2001) Biologicals. 29:123-32.
[76] Gennaro (2000) Remington: The Science and Practice of Pharmacy. 20th edition, ISBN: 0683306472.
[77] Banzhoff (2000) Immunology Letters 71:91-96.
[78] Nony et al. (2001) Vaccine 27:3645-51.
[185] Potter & Oxford (1979) Br Med Bull 35: 69-75.
[186] Greenbaum et al. (2004) Vaccine 22:2566-77.
[187] Zurbriggen et al. (2003) Expert Rev Vaccines 2:295-304.
[188] Piascik (2003) J Am Pharm Assoc (Wash DC). 43:728-30.
[189] Mann et al. (2004) Vaccine 22:2425-9.
[190] Halperin et al. (1979) Am J Public Health 69:1247-50.
[191] Herbert et al. (1979) J Infect Dis 140:234-8.
[192] Chen et al. (2003) Vaccine 21:2830-6.
[193] Gambaryan et al. (1997) Virology 232:345-50.

## Claims

1. A process for manufacturing a vaccine comprising steps of:
(i) isolating an influenza virus from a patient sample, comprising a step in which the patient sample is incubated with a MDCK cell, wherein the MDCK cell is growing in a serum-free suspension culture, and
(ii) manufacturing a vaccine from the influenza virus isolated in step (i),
wherein the influenza virus is not grown in eggs before being exposed to MDCK cells, and wherein after isolation, the virus is not subsequently grown in eggs.

2. The process of claim 1, wherein the MDCK cell is growing in a protein-free suspension culture.

3. The process of claim 1 or claim 2, wherein:
(a) the MDCK cell is non-tumorigenic;
(b) the MDCK cell is not provided with an overlay medium; and/or
(c) the MDCK cell is MDCK 33016.

4. The process of any preceding claim, wherein the influenza virus that is isolated in step (i) is selected from an H1N1 or H3N2 influenza A strain or an influenza B strain.

5. The process of any preceding claim, wherein after isolation, the virus is passaged and/or grown in MDCK cells.

6. The process of any preceding claim, wherein after isolation, the virus is passaged and/or grown in non-MDCK cells or in another substrate.

7. The process of any preceding claim, wherein the vaccine comprises hemagglutinin (HA) antigens from 2, 3, 4, or more influenza virus strains, wherein the strains are influenza A virus and/or influenza B virus.

8. The process of any preceding claim, wherein the influenza virus strain that is isolated in step (i) has a binding preference for oligosaccharides with a Sia(α2,6)Gal terminal disaccharide compared to oligosaccharides with a Sia(α2,3)Gal terminal disaccharide.

9. The process of any preceding claim, wherein the influenza virus includes hemagglutinin with a binding preference for oligosaccharides with a Sia(α2,6)Gal terminal disaccharide compared to oligosaccharides with a Sia(α2,3)Gal terminal disaccharide.

10. A process for preparing an influenza virus antigen for use in a vaccine, comprising steps of: (i) receiving an influenza virus isolated from a patient sample by a method comprising a step in which the patient sample is incubated with a MDCK cell, wherein the MDCK cell is growing in a serum-free suspension culture; (ii) infecting a cell line with this influenza virus; and (iii) culturing the infected cells from step (ii) in order to produce influenza virus, wherein the influenza virus is not grown in eggs before being exposed to MDCK cells, and wherein after isolation, the virus is not subsequently grown in eggs.

11. The process of claim 10, wherein the influenza virus in step (i) includes hemagglutinin with a binding preference for oligosaccharides with a Sia(α2,6)Gal terminal disaccharide compared to oligosaccharides with a Sia(α2,3)Gal terminal disaccharide.

12. The process of claim 10 or claim 11, wherein the influenza virus in step (i) has glycoproteins with a different glycosylation pattern from egg-derived viruses.

13. The process of any one of claims 10 to 12, wherein the influenza virus has never been propagated on a substrate growing in a serum-containing medium.

14. The process of any one of claims 10 to 13, wherein the process comprises (iv) manufacturing a vaccine, further wherein the vaccine comprises hemagglutinin (HA) antigens from 2, 3, 4, or more influenza virus strains, and the strains are influenza A virus and/or influenza B virus.

## Patentansprüche

1. Verfahren zur Erzeugung eines Impfstoffs, umfassend die Schritte:
(i) Isolieren eines Influenzavirus aus einer Patientenprobe, umfassend einen Schritt, bei dem die Patientenprobe mit einer MDCK-Zelle inkubiert wird, wobei die MDCK-Zelle in einer serumfreien Suspensionskultur wächst, und
(ii) Erzeugen eines Impfstoffs aus dem in Schritt (i) isolierten Influenzavirus,
wobei das Influenzavirus vor dem in Kontakt Bringen mit MDCK-Zellen nicht in Eiern kultiviert wird und wobei nach der Isolierung das Virus anschließend nicht in Eiern kultiviert wird.

2. Verfahren nach Anspruch 1, wobei die MDCK-Zelle in einer proteinfreien Suspensionskultur wächst.

3. Verfahren nach Anspruch 1 oder Anspruch 2, wobei:
(a) die MDCK-Zelle nicht tumorigen ist;
(b) die MDCK-Zelle nicht mit einem Overlay-Medium versehen ist; und/oder
(c) es sich bei der MDCK-Zelle um MDCK 33016 handelt.

4. Verfahren nach einem der vorhergehenden Anspruch, wobei das Influenzavirus, das in Schritt (i) isoliert wird, aus einem H1N1- oder H3N2-Influenza-A-Stamm oder einem Influenza-B-Stamm ausgewählt ist.

5. Verfahren nach einem vorhergehenden Anspruch, wobei nach der Isolierung das Virus in MDCK-Zellen passagiert und/oder kultiviert wird.

6. Verfahren nach einem vorhergehenden Anspruch, wobei nach der Isolierung das Virus in Nicht-MDCK-Zellen oder in einem anderen Substrat passagiert und/oder kultiviert wird.

7. Verfahren nach einem vorhergehenden Anspruch, wobei der Impfstoff Hämagglutinin (HA) -Antigene aus 2, 3, 4 oder mehr Influenzavirus-Stämmen umfasst, wobei es sich bei den Stämmen um Influenza-A-Virus und/oder Influenza-B-Virus handelt.

8. Verfahren nach einem vorhergehenden Anspruch, wobei der Influenzavirus-Stamm, der in Schritt (i) isoliert wird, eine Bindungspräferenz für Oligosaccharide mit einem endständigen Sia(α2,6)Gal-Disaccharid gegenüber Oligosacchariden mit einem endständigen Sia(a2,3)Gal-Disaccharid aufweist.

9. Verfahren nach einem vorhergehenden Anspruch, wobei das Influenzavirus Hämagglutinin mit einer Bindungspräferenz für Oligosaccharide mit einem endständigen Sia(α2,6)Gal-Disaccharid gegenüber Oligosacchariden mit einem endständigen Sia(α2,3)Gal-Disaccharid enthält.

10. Verfahren zur Herstellung eines Influenzavirus-Antigens zur Verwendung in einem Impfstoff, umfassend die Schritte: (i) Erhalten eines Influenzavirus, das aus einer Patientenprobe mit einem Verfahren isoliert wird, das einen Schritt umfasst, bei dem die Patientenprobe mit einer MDCK-Zelle inkubiert wird, wobei die MDCK-Zelle in einer serumfreien Suspensionskultur wächst; (ii) Infizieren einer Zelllinie mit diesem Influenzavirus; und (iii) Kultivieren der infizierten Zellen aus Schritt (ii), um Influenzavirus zu produzieren, wobei das Influenzavirus vor dem in Kontakt Bringen mit MDCK-Zellen nicht in Eiern kultiviert wird und wobei nach der Isolierung das Virus anschließend nicht in Eiern kultiviert wird.

11. Verfahren nach Anspruch 10, wobei das Influenzavirus in Schritt (i) Hämagglutinin mit einer Bindungspräferenz für Oligosaccharide mit einem endständigen Sia(α2,6)Gal-Disaccharid gegenüber Oligosacchariden mit einem endständigen Sia(α2,3)Gal-Disaccharid enthält.

12. Verfahren nach Anspruch 10 oder Anspruch 11, wobei das Influenzavirus in Schritt (i) Glykoproteine mit einem anderen Glykosylierungsmuster als bei aus Eiern gewonnenen Viren aufweist.

13. Verfahren nach einem der Ansprüche 10 bis 12, wobei das Influenzavirus nie auf einem Substrat propagiert wurde, das in einem serumhaltigen Medium wächst.

14. Verfahren nach einem der Ansprüche 10 bis 13, wobei das Verfahren (iv) Erzeugen eines Impfstoffs umfasst, weiter wobei der Impfstoff Hämagglutinin(HA)-Antigene aus 2, 3, 4 oder mehr Influenzavirus-Stämmen umfasst und es sich bei den Stämmen um Influenza-A-Virus und/oder Influenza-B-Virus handelt.

## Revendications

1. Procédé de fabrication d'un vaccin comprenant les étapes de :
(i) isolement d'un virus de la grippe à partir d'un échantillon de patient, comprenant une étape dans laquelle l'échantillon de patient est incubé avec une cellule MDCK, la cellule MDCK croissant dans une culture en suspension sans sérum, et
(ii) fabrication d'un vaccin à partir du virus de la grippe isolé à l'étape (i),
dans lequel le virus de la grippe n'est pas cultivé dans des œufs avant d'être exposé aux cellules MDCK, et dans lequel, après isolement, le virus n'est pas cultivé ultérieurement dans des œufs.

2. Procédé selon la revendication 1, dans lequel la cellule MDCK croît dans une culture en suspension sans protéines.

3. Procédé selon la revendication 1 ou la revendication 2, dans lequel :
(a) la cellule MDCK n'est pas tumorigène ;
(b) la cellule MDCK n'est pas dotée d'un milieu de recouvrement ; et/ou
(c) la cellule MDCK est MDCK 33016.

4. Procédé selon l'une quelconque des revendications précédentes, dans lequel le virus de la grippe qui est isolé à l'étape (i) est choisi parmi une souche de grippe A H1N1 ou H3N2 ou une souche de grippe B.

5. Procédé selon l'une quelconque des revendications précédentes, dans lequel, après isolement, le virus est soumis à un passage et/ou une culture dans des cellules MDCK.

6. Procédé selon l'une quelconque des revendications précédentes, dans lequel, après isolement, le virus est soumis à un passage et/ou une culture dans des cellules non MDCK ou dans un autre substrat.

7. Procédé selon l'une quelconque des revendications précédentes, dans lequel le vaccin comprend des antigènes d'hémagglutinine (HA) provenant de 2, 3, 4 ou plus de 4 souches de virus de la grippe, les souches étant un virus de la grippe A et/ou un virus de la grippe B.

8. Procédé selon l'une quelconque des revendications précédentes, dans lequel la souche de virus de la grippe qui est isolée à l'étape (i) a une préférence de liaison pour les oligosaccharides comportant un disaccharide terminal Sia(α2,6)Gal par rapport aux oligosaccharides avec un disaccharide terminal Sia(α2,3)Gal.

9. Procédé selon l'une quelconque des revendications précédentes, dans lequel le virus de la grippe comprend de l'hémagglutinine ayant une préférence de liaison pour les oligosaccharides avec un disaccharide terminal Sia(α2,6)Gal par rapport aux oligosaccharides avec un disaccharide terminal Sia(α2,3)Gal.

10. Procédé de préparation d'un antigène de virus de la grippe destiné à être utilisé dans un vaccin, comprenant les étapes de : (i) réception d'un virus de la grippe isolé à partir d'un échantillon de patient par un procédé comprenant une étape dans laquelle l'échantillon de patient est incubé avec une cellule MDCK, la cellule MDCK croissant dans une culture en suspension sans sérum ; (ii) infection d'une lignée cellulaire avec ce virus de la grippe ; et (iii) culture des cellules infectées issues de l'étape (ii) afin de produire un virus de la grippe, le virus de la grippe n'étant pas cultivé dans des œufs avant d'être exposé à des cellules MDCK, et après isolement, le virus n'étant pas cultivé ultérieurement dans des œufs.

11. Procédé selon la revendication 10, dans lequel le virus de la grippe dans l'étape (i) comprend de l'hémagglutinine ayant une préférence de liaison pour les oligosaccharides avec un disaccharide terminal Sia(α2,6)Gal par rapport aux oligosaccharides avec un disaccharide terminal Sia(α2,3)Gal.

12. Procédé selon la revendication 10 ou la revendication 11, dans lequel le virus de la grippe à l'étape (i) comporte des glycoprotéines ayant un profil de glycosylation différent de celui de virus dérivés d'œufs.

13. Procédé selon l'une quelconque des revendications 10 à 12, dans lequel le virus de la grippe n'a jamais été propagé sur un substrat croissant dans un milieu contenant du sérum.

14. Procédé selon l'une quelconque des revendications 10 à 13, le procédé comprenant (iv) la fabrication d'un vaccin, le vaccin comprenant en outre des antigènes d'hémagglutinine (HA) provenant de 2, 3, 4 ou plus de 4 souches de virus de la grippe, et les souches sont un virus de la grippe A et/ou un virus de la grippe B.
